# EUROPEAN PATENT APPLICATION

(11) **EP 3 882 748 A1**
(43) Date of publication of application: **22.09.2021**
(21) Application number: 21152739.5
(22) Date of filing: 21.01.2021
(51) Int. Cl.: G06F 3/0338, A61L 2/00

(54) **INDIRECT TOUCH APPARATUS**

(30) Priority: 20.03.2020 KR 20200034305; 02.11.2020 KR 20200144119
(71) Applicant: Kim, Soonil, Seoul (KR)
(72) Inventor: Kim, Soonil, Seoul (KR)
(74) Representative: Fortuna, Jevgenijs

(57) **Abstract**

An object of the present invention is to provide an indirect touch apparatus capable of preventing contamination of a hand or a pocket. To this end, the indirect touch apparatus of the present invention is an indirect touch apparatus for touching indirectly a touch object and includes a housing having a draw-in/out guide path concaved in a longitudinal direction; a touch part slidably provided on the draw-in/out guide path and drawn out to come in contact with the touch object; and pressing part drawing in/out the touch part to/from the housing.

## Description

### Technical Field

The present invention relates to an indirect touch apparatus.

### Background Art

In general, indirect touch is an act of indirectly contacting a touch object using a separate device without directly touching a touch object by a user's hand that is contaminated or expected to be contaminated.

An indirect touch apparatus for such indirect touch may have a rod shape. For example, when the touch object is a button of an elevator that is used by an unspecified number of people, the user may take out a bar-shaped indirect touch apparatus from a pocket to press the button indirectly through the indirect touch apparatus.

However, when such a rod-shaped indirect touch apparatus comes into contact with a touch object contaminated with viruses or bacteria, the contacted part may be first contaminated, and if the indirect touch apparatus enters the user's pocket in the contaminated state, there is a problem that the pocket and the like are second contaminated.

In addition, there is a problem that such a bar-shaped indirect touch apparatus cannot be used for a capacitive touch object such as a capacitive touch button or a capacitive touch screen. For example, when an elevator button is a capacitive button that operates by recognizing a change in capacitance, there is a problem that the indirect touch apparatus is not operated when used as it is.

### Disclosure

### Technical Problem

An object of the present invention is to provide an indirect touch apparatus capable of preventing contamination of a hand or a pocket.

Another object of the present invention is to provide an indirect touch apparatus capable of being used for a capacitive touch object.

Yet another object of the present invention is to provide an indirect touch apparatus capable of eradicating a contaminant of a part in contact with a touch object as much as possible.

### Technical Solution

In order to achieve the above objects, an indirect touch apparatus according to an embodiment of the present invention for indirectly touching a touch object, including: a housing having a draw-in/out guide path concaved in a longitudinal direction; a touch part slidably provided on the draw-in/out guide path and drawn out to be in contact with the touch object; and a pressing part for drawing in/out the touch part to/from the housing.

The indirect touch apparatus according to the embodiment of the present invention described above may further include a cover part provided at a front end of the housing and selectively covering the end of the draw-in/out guide path.

When provided at the front end of the housing, the cover part may open the end of the draw-in/out guide path using a draw-out force of the touch part while the touch part is drawn out by the pressing part and close the end of the draw-in/out guide path while the touch part is drawn in by the pressing part.

As an example, the cover part may include a cover member made of an elastic material covering an end of the draw-in/out guide path; and a slit formed in the middle of the cover member and is opened while the touch part is drawn out and retracted while the touch part is drawn in.

As another example, the cover part may include a cover member provided rotatably at the end of the draw-in/out guide path; a pinion provided on a rotation shaft of the cover member; and a rack provided on one surface of the touch part so as to be geared to the pinion.

The number of gear teeth of the rack may be determined such that the cover member is rotated by 90° or more or 180° or more when the cover member is opened by being geared to the pinion.

The cover part may further include a torsion coil spring which is provided on the rotation shaft of the cover member, and applies a rotational force to the cover member in a closed direction when the draw-in of the touch part is completed and the rack is disengaged from the pinion.

As yet another example, the cover part may include a cover member rotatably provided below the front end of the housing; a link member having one end rotatably provided on the inner surface of the cover member; a guide groove elongated on the inner side of the housing in a longitudinal direction and provided to communicate with the draw-in/out guide path; a front-rear moving rail which is provided to be movable front and rear in the guide groove, has the other end of the link member rotatably provided at a front end, and has a front-rear guide hole elongated in a longitudinal direction; and a pulling protrusion that is provided to protrude from the side of the touch part and moves along the front-rear guide hole while the touch part is drawn in and out by the pressing part, and pulls the front-rear moving rail back when the touch part is drawn in.

The cover part may further include an elastic body which is provided between a rear end of the front-rear moving rail and a rear end of the guide groove and applies a force pushing forward to the front-rear moving rail; and a position maintaining part that maintains the position of the link member even if the front-rear moving rail is pushed forward by the elastic body.

The position maintaining part may include a pushing guide hole elongated in the link member in a longitudinal direction; and a rotation shaft which is provided at a front end of the front-rear moving rail, passes through the pushing guide hole, guides the rotation of the link member, and moves along the pushing guide hole when the front-rear moving rail is pushed forward.

As still another example, the cover part may open the end of the draw-in/out guide path in advance with a time lag before coming into contact with the touch part while the touch part is moved in a direction to be drawn out by the pressing part and close the end of the draw-in/out guide path with a time lag after the touch part is drawn in.

The cover part may include a reference member fixed to the inner front end of the housing; a cover member rotatably provided at the front end of the reference member; a moving member that is provided between the touch part and the reference member, is selectively connected to the touch part, and moves front and rear together with the touch part when connected to the touch part, wherein the moving distance is limited by the reference member to have the time lag; a motion switching part for rotating the cover member forward and backward by the front and rear movement of the moving member.

The motion switching part may include a cover hinge rotatably connecting the cover member to a front end of the reference member; a locking groove formed at an edge of the cover member and provided to be eccentric with the cover hinge; and a power transmission protrusion that is provided at the front end of the moving member and selectively locked to the locking groove while the moving member moves front and rear to apply a forward and reverse rotational force to the cover member.

The moving member may be selectively connected to the touch part through a connection part.

The connection part may include a connection groove provided on the touch part; and a connection protrusion that is provided in the moving member so as to be locked or removed from the connection groove while the touch part moves front and rear.

The pressing part may include a knob guide hole formed to penetrate through one surface of the housing and elongated in a longitudinal direction thereof to limit a draw-in/out distance; and a pressing knob having one end exposed to the outside through the knob guide hole and the other end provided on the touch part.

The pressing part may be made of an antimicrobial material itself, or the antimicrobial material may be contained therein or coated on the surface thereof.

The pressing part may be made of a conductive material itself, or the conductive material may be contained therein or further coated on the surface thereof.

The touch part may be made of an antimicrobial material itself, or the antimicrobial material may be contained therein or coated on the surface thereof.

The touch part may be made of a conductive material itself, or the conductive material may be contained therein or further coated on the surface thereof.

The housing may be made of an antimicrobial material itself, or the antimicrobial material may be contained therein or coated on the surface thereof.

The housing may be made of a conductive material itself, or the conductive material may be contained therein or coated on the surface thereof.

The touch part, the housing, or the pressing part may include any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent and a natural antimicrobial agent. Alternatively, a small ultraviolet light generating device may be installed or an antimicrobial agent may be filled inside the housing. For reference, the antimicrobial includes all bacteria, fungi, viruses, protozoa, and the like, and the antibacterial agent may be referred to as only a substance targeting bacteria as a narrow meaning or may be used as an antimicrobial agent as a broad meaning.

The touch part may include a sliding member that slides along the draw-in/out guide path and has a conductive material contained therein or coated on an outer surface thereof; and a touch member made of a soft material that is provided at the end of the sliding member and is provided at a distance from the end of the sliding member in a longitudinal direction and has a conductive material contained therein or coated on an outer surface thereof.

The touch part may include a pressure-resistant member made of a hard material that is provided at the end of the sliding member, is provided to be surrounded by the touch member, is provided at a distance from the touch member in a longitudinal direction, and has a convex end.

In addition to the conductive material, the sliding member may contain an antimicrobial material therein or the antimicrobial material may be coated on the outer surface thereof.

The conductive and antimicrobial materials of the sliding member may include any one of carbon black, carbon nanotube, carbon fiber, graphite, graphene, a conductive polymer material, an antistatic agent, and a metal, and any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent, and a natural antimicrobial agent. The inorganic antimicrobial agent is not limited only to the substances listed above. Alternatively, a small ultraviolet light generating device may be installed or an antimicrobial agent may be filled inside the housing.

The touch member may be formed of any one or at least two of metal, plastic, thermoplastic elastomer, rubber, silicone, metal mesh, and fiber. In the touch part, any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent, a natural antimicrobial agent may be further contained therein or further coated on the outer surface thereof. The inorganic antimicrobial agent is not limited only to the substances listed above.

The touch member may be in contact with an area of 1 mm² or more, preferably 4 mm² or more, and more preferably 9 mm² or more.

### Advantageous Effects

As described above, the indirect touch apparatus according to the embodiment of the present invention may have the following effects.

According to the embodiment of the present invention, as an indirect touch apparatus for indirectly touching a touch object, since there is provided a technical configuration including a housing, a touch part, and a pressing part, a user draws out the touch part from the housing using the pressing part to come into contact with a touch object or draws the touch part into the housing using the pressing part again after coming into contact with a touch object while the force is applied, thereby preventing contamination of a hand, a pocket, or the like while the touch part is drawn into the housing even if a contaminant of the touch object is stained on the touch part.

Further, since there is provided a technical configuration including a cover part, the contaminant of the touch object stained on the touch part may be prevented from being discharged to the outside through the draw-in/out guide path or prevented from coming into contact with a user's body, clothes, and belongings, thereby more efficiently preventing contamination of the hand, the pocket, or the like.

Further, since there is provided a technical configuration in which a cover part includes, as an example, a cover member and a slit, while the touch part is drawn out, the slit of the cover member made of an elastic material is opened so that the touch part may be smoothly drawn out, and when the draw-in of the touch part is completed, the slit is closed due to the contraction of the cover member to block the draw-in/out guide path, thereby configuring the cover part with a relatively simple configuration.

Further, since there is provided a technical configuration in which a cover part includes, as another example, a cover member, a pinion, and a rack, while the touch part is drawn out, the rack provided therein is drawn out together to rotate the pinion in the direction in which the cover member is opened. On the contrary, while the touch part is drawn in, the pinion may be rotated in the direction in which the cover member is closed, so that the cover member may have improved durability as compared with the cover part of the example described above which is opened with respect to the slit.

Further, since there is provided a technical configuration in which the number of gear teeth of the rack is determined such that the cover member rotates approximately 90° or more or approximately 180° or more when the cover member is opened while geared to the pinion, when the cover member is opened, the cover member does not protrude in the longitudinal direction of the touch part, but may be vertical to the longitudinal direction of the housing or adjacent to the outer surface of the housing, thereby preventing the cover member from interfering with the touch object while the touch part touches the touch object.

Further, since there is provided a technical configuration in which the cover part of the aforementioned another example further includes a torsion coil spring, even if the gear engagement between the rack and the pinion is momentarily released after the touch part is drawn in and the cover member is closed, the rotational force of the torsion coil spring may be applied in the closing direction, thereby increasing the adhesion of the cover member to the end of the housing.

Further, since there is provided a technical configuration in which while a cover part is provided at the front end of the housing, the cover part opens the end of the draw-in/out guide path in advance with a time lag before coming into contact with the touch part while the touch part is moved in a direction to be drawn out by the pressing part and closes the end of the draw-in/out guide path with a time lag after the touch part is drawn in, since the end of the touch part does not directly contact the cover part while the cover part is opened or closed, there is no interference between the touch part and the cover part to improve the durability of the touch part and the cover part. In addition, since there is no friction with the touch part, the cover part may be opened or closed smoothly, and the cover part may always be kept in a clean state even if a contaminant is stained on the end of the touch part.

Further, since there is provided a technical configuration in which a pressing part includes a knob guide hole and a pressing knob, when the user pushes the pressing knob with a finger or the like, the pressing knob is moved along the knob guide hole and then stops at the end of the knob guide hole to limit the draw in/out distance of the touch part, thereby preventing the touch part from being drawn out too much and minimizing the touch part from being bent or broken even if the touch part strongly presses the touch object.

Further, since there is provided a technical configuration in which a touch part is made of an antimicrobial material itself or a conductive material itself, or the antimicrobial and conductive materials are contained therein or coated on the surface thereof, when the touch part comes into contact with a capacitive touch object (such as a capacitive touch button or a capacitive touch screen), the touch part made of the conductive material may be electrically connected with the hand as a type of conductor through the sliding member, the housing, the pressing part, or the like. When the touch part closely approaches an electrode of the capacitive touch object, electric charges may be collected between the electrode and the touch part and as the charges are collected in this way, it is possible to measure the electrostatic capacity or electric capacity between the electrode and the touch part, and by using this phenomenon, the touch of the touch part may be indirectly detected. Furthermore, as the touch part has an antimicrobial material, the antimicrobial material may eradicate the contaminant stained on the touch object from the touch part as much as possible.

Further, since there is provided a technical configuration in which in the housing, an antimicrobial material is contained therein or coated on the surface thereof, while the contaminant stained on the touch part is drawn into the draw-in/out guide path of the housing, even if the contaminant is transmitted to the inner surface of the draw-in/out guide path through the touch part, it is possible to prevent the contaminant from remaining on the inner surface of the draw-in/out guide pat as much as possible.

Further, since there is provided a technical configuration in which the touch part includes a sliding member and a touch member, the touch member made of a soft material may come into contact with the touch object, thereby preventing damage to the touch object, such as scratching and the like, and it possible to increase the touch recognition rate of a touch object to be operated by more closely contacting a capacitive touch screen and a capacitive touch button.

Further, since there is provided a technical configuration in which the touch part further includes a pressure-resistant member, when the touch part strongly presses the touch object, even if the touch member is crushed, the touch part may apply sufficient pressure to the touch object (for example, a pressing button to operate only when pressed) while the pressure-resistant member supports the pressing force.

### Description of Drawings

FIG. 1 is a diagram schematically illustrating an indirect touch apparatus according to a first embodiment of the present invention.
FIG. 2 is a diagram schematically illustrating a state in which a touch part of the indirect touch apparatus of FIG. 1 is drawn out.
FIG. 3 is a diagram schematically illustrating an indirect touch apparatus according to a second embodiment of the present invention.
FIG. 4 is a diagram schematically illustrating a state in which a touch part of the indirect touch apparatus of FIG. 3 is drawn out.
FIG. 5 is a diagram schematically illustrating an indirect touch apparatus according to a third embodiment of the present invention.
FIG. 6 is a diagram schematically illustrating a state in which a touch part of the indirect touch apparatus of FIG. 5 is drawn out.
FIG. 7 is a diagram schematically illustrating an indirect touch apparatus according to a fourth embodiment of the present invention.
FIG. 8 is a diagram schematically illustrating a state in which a touch part of the indirect touch apparatus of FIG. 7 is drawn out to push a cover member.
FIG. 9 is a diagram schematically illustrating a state in which the touch part of the indirect touch apparatus of FIG. 8 is fully drawn out.
FIG. 10 is a diagram schematically illustrating a state in which a front-rear moving rail is pulled by a pulling protrusion while the touch part of the indirect touch apparatus of FIG. 9 is drawn in through the state of FIG. 8.
FIG. 11 is a diagram schematically illustrating an indirect touch apparatus according to a fifth embodiment of the present invention.
FIG. 12 is a diagram of the indirect touch apparatus of FIG. 11 taken along line XII-XII.
FIG. 13 is a side view illustrating the indirect touch apparatus of FIG. 11 from which a housing is removed.
FIG. 14 is a side view illustrating the indirect touch apparatus of FIG. 13 from which a reference member is removed.
FIG. 15 is a diagram schematically illustrating a state in which a cover part is opened before the touch part comes into contact with the cover part while the touch part is moved in a direction to be drawn out by a pressing part in the indirect touch apparatus of FIG. 14.
FIG. 16 is a diagram schematically illustrating a state in which the touch part is fully drawn out by the pressing part in the indirect touch apparatus of FIG. 15.
FIG. 17 is a diagram schematically illustrating a state in which the touch part is fully drawn out in the indirect touch apparatus of FIG. 12.

### Modes for the Invention

Hereinafter, embodiments of the present invention will be described in detail with reference to the accompanying drawings so as to easily implement those with ordinary skill in the art to which the present invention pertains. However, the present invention may be embodied in many different forms and are limited to embodiments described herein.

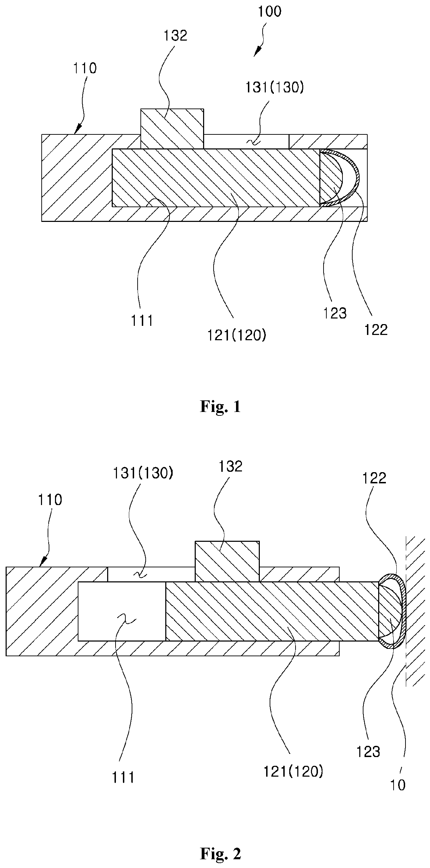

FIG. 1 is a diagram schematically illustrating an indirect touch apparatus according to a first embodiment of the present invention and FIG. 2 is a diagram schematically illustrating a state in which a touch part of the indirect touch apparatus of FIG. 1 is drawn out.

The indirect touch apparatus 100 according to the first embodiment of the present invention, as illustrated in FIGS. 1 and 2, is an indirect touch apparatus for indirectly touching a touch object 10 to be touched, and includes a housing 110, a touch part 120 and a pressing part 130. Hereinafter, respective components will be described in detail with reference to FIGS. 1 and 2.

The housing 110 is a component constituting the exterior of the indirect touch apparatus 100 of the present invention. This housing 110, as illustrated in FIGS. 1 and 2, may have a draw-in/out guide path 111 concaved into the housing from its end along a longitudinal direction. For example, although not illustrated, the housing 110 may have an external shape such as a circular shape, a rectangular parallelepiped shape, or an ellipse shape.

The touch part 120 is a component for substantially touching the touch object 10 (including not only a simple contact but also an action pressing by applying a force) instead of a user's hand. As illustrated in FIGS. 1 and 2, the touch part 120 may be provided slidably in the draw-in/out guide path 111 and may be drawn out to come into contact with the touch object 10.

The pressing part 130 is a component for drawing in/out the touch part 120 in the housing 110. For example, such a pressing part 130 may include a knob guide hole 131 and a pressing knob 132 as illustrated in FIGS. 1 and 2. The knob guide hole 131 may be formed to penetrate through one surface of the housing 110 and may be elongated in a longitudinal direction thereof, and the pressing knob 132 may have one end exposed to the outside through the knob guide hole 131 and the other end provided on the touch part 120. Accordingly, when the user pushes the pressing knob 132 with a finger or the like in the longitudinal direction of the housing 110, the pressing knob 132 is moved along the knob guide hole 131 and the touch pat 120 may be drawn out to the outside of the housing 110. Further, the knob guide hole 131, as illustrated in FIG. 2, may limit the draw in/out distance of the touch part 120 by limiting the moving distance of the pressing knob 132. That is, when the user pushes the pressing knob 132, the pressing knob 132 moves along the knob guide hole 131 and then stops at the end of the knob guide hole 131 to limit the draw in/out distance of the touch part 120, thereby preventing the touch part 120 from being drawn out too much and minimizing the touch part 120 from being bent or broken even if the touch part 120 strongly presses the touch object 10.

Therefore, since the above components are provided, the user draws out the touch part 120 from the housing 110 using the pressing part 130 to come into contact with the touch object 10 or contacts the touch part 120 with the touch object 10 in a state where a force is applied and then draws in the touch part 120 to the housing 110 using the pressing part 130, thereby preventing contamination of a hand or a pocket while the touch part 120 is drawn into the housing 110 even if the touch part 120 is stained with a contaminant of the touch object 10.

Hereinafter, referring back to FIGS. 1 and 2, the housing 110, the touch part 120, and the pressing part 130 will be described in more detail.

The housing 110 may be made of an antimicrobial material itself, or the antimicrobial material may be contained therein or coated on the surface thereof. For example, the antimicrobial material may include any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent and a natural antimicrobial agent. Alternatively, a small ultraviolet light generating device may be installed or an antimicrobial agent may be filled inside the housing.

For reference, in the case where the housing 110 is a plastic injection product, as an example, when a molding material is mixed before melting, an antibacterial material is added to a plastic resin and mixed together, so that the antimicrobial material may be contained in the housing 110. As another example, the housing 110 is molded into a plastic injection product, and then the antimicrobial material may be coated on the outer surface thereof.

Therefore, while the contaminant stained on the touch part 120 is drawn into the draw-in/out guide path 111 of the housing 110, the contaminant transmitted to the inner surface of the draw-in/out guide path 111 through the touch part 120 may be eradicated as much as possible by the antibacterial material to prevent the contaminant from remaining on the inner surface of the draw-in/out guide path 111 as much as possible.

The touch part 120 may be made of a conductive or antimicrobial material itself, or the conductive or antimicrobial material may be contained therein or coated on the surface thereof. For example, the conductive and antimicrobial materials may include any one of carbon black, carbon nanotube, carbon fiber, graphite, graphene, a conductive polymer material, an antistatic agent, and a metal, and any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent, and a natural antimicrobial agent. Alternatively, a small ultraviolet light generating device may be installed or an antimicrobial agent may be filled inside the touch part. For reference, in the case where the touch part 120 is made of plastic or elastomer, as an example, when a molding material is mixed, the conductive and antimicrobial materials are added and mixed together, so that the conductive and antimicrobial materials may be contained in the touch part 120. As another example, the touch part 120 is molded and then the conductive and antimicrobial materials may also be coated on the surface thereof.

The pressing part 130 may be made of a conductive or antimicrobial material itself, or the conductive or antimicrobial material may be contained therein or coated on the surface thereof. For example, the conductive and antimicrobial materials may include any one of carbon black, carbon nanotube, carbon fiber, graphite, graphene, a conductive polymer material, an antistatic agent, and a metal, and any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent, and a natural antimicrobial agent. Alternatively, a small ultraviolet light generating device may be installed or an antimicrobial agent may be filled inside the pressing part. For reference, in the case where the pressing part 130 is made of plastic or elastomer, as an example, when a molding material is mixed, the conductive and antimicrobial materials are added and mixed together, so that the conductive and antimicrobial materials may be contained in the pressing part 130. As another example, the pressing part 130 is molded and then the conductive and antimicrobial materials may also be coated on the surface thereof.

Furthermore, these conductive and antimicrobial materials may be used for the housing 110.

Therefore, when the touch part 120 comes into contact with a capacitive touch object (such as a capacitive touch button or a capacitive touch screen) (see 10), the touch part 120 made of the conductive material may be electrically connected with the hand by the housing 110 or the pressing part 130. When the touch part 120 closely approaches an electrode (not illustrated) of the capacitive touch object 10, electric charges may be collected between the electrode (not illustrated) and the touch part 120. As the charges are collected in this way, it is possible to measure the electrostatic capacity or electric capacity between the electrode (not illustrated) and the touch part 120, and by using this phenomenon, the touch of the touch part 120 may be indirectly detected. Furthermore, as the touch part 120 has an antimicrobial material, the antimicrobial material may eradicate the contaminant stained on the touch object 10 from the touch part 120 as much as possible.

Further, the touch part 120 may include a sliding member 121 and a touch member 122 as illustrated in FIGS. 1 and 2. The sliding member 121 may slide along a draw-in/out guide path 111, and as described above, the conductive material may be contained therein or coated on the surface thereof. The touch member 122 may be provided at an end of the sliding member 121 and may be provided at a distance from the end of the sliding member 121 in the longitudinal direction, and may be made of a conductive material itself or the conductive material may be contained therein, or coated on the surface thereof.

For example, in the sliding member 121, in addition to the conductive material as mentioned in the touch part 120, the antimicrobial material may be contained therein or may be coated on the surface thereof. The conductive and antimicrobial materials of the sliding member 121 may include any one of carbon black, carbon nanotube, carbon fiber, graphite, graphene, a conductive polymer material, an antistatic agent, and a metal, and any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent, and a natural antimicrobial agent. Alternatively, a small ultraviolet light generating device may be installed or an antimicrobial agent may be filled inside the sliding member.

Further, the touch member 122 may be formed of any one of metal, plastic, thermoplastic elastomer, rubber, silicone, metal mesh, and fiber or a combination of at least two thereof. Further, in the touch part 122, any one or at least one of an inorganic antimicrobial agent, for example, a metal such as copper, silver, zirconium, zinc, or gold, a compound of the metal and other elements, or a material in which the metal is adsorbed or bound to as a carrier such as zeolite or glass; an organic antimicrobial agent, a natural antimicrobial agent may be further contained therein or further coated on the outer surface thereof. Here, the inorganic antimicrobial agent is not limited only to the substances listed above.

In addition, in order to increase the touch recognition rate for the touch object 10, the touch member 122 may have an area of 4 mm² or more, preferably 9 mm² or more, when in contact with the touch object 10.

Further, the touch part 120 may further include a pressure-resistant member 123 as illustrated in FIGS. 1 and 2. The pressure-resistant member 123 may be made of a hard material, may be provided at the end of the sliding member 121, but may be provided so as to be surrounded by the touch member 122, and spaced apart from the touch member 122 in the longitudinal direction. Therefore, when the touch part 120 strongly presses the touch object 10, even if the touch member 122 is crushed, the touch part 120 may apply sufficient pressure to the touch object 10 (for example, a pressing button to operate only when pressed) while the pressure-resistant member 123 supports the pressing force.

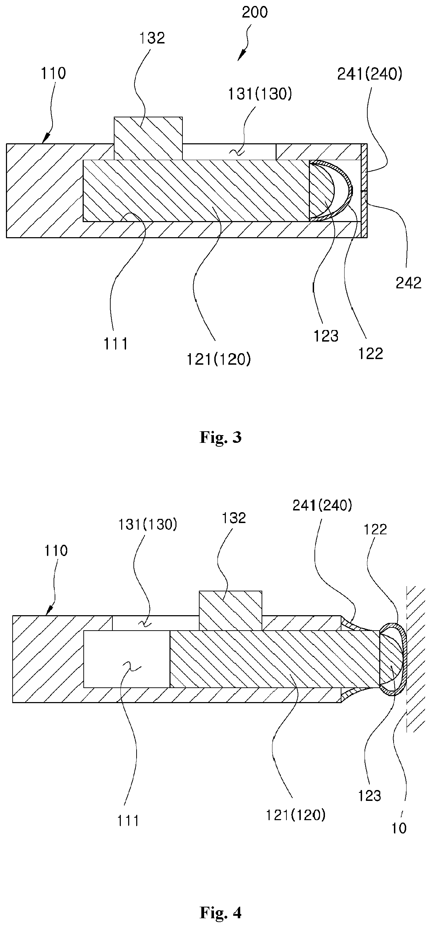

Hereinafter, a direct touch apparatus 200 according to a second embodiment of the present invention will be described with reference to FIGS. 3 and 4.

FIG. 3 is a diagram schematically illustrating an indirect touch apparatus according to a second embodiment of the present invention and FIG. 4 is a diagram schematically illustrating a state in which a touch part of the indirect touch apparatus of FIG. 3 is drawn out.

The indirect touch apparatus 200 according to the second embodiment of the present invention is the same as that of the first embodiment of the present invention described above except that a cover part 240 is further included, as illustrated in FIGS. 3 and 4, and thus, hereinafter, the cover part 240 will be mainly described.

The cover part 240 is a component for preventing a contaminant of the touch object 10 stained on the touch part 120 from being discharged to the outside through a draw-in/out guide path 111. This cover part 240, as illustrated in FIGS. 3 and 4, may be provided at the front end of the housing 110, and may selectively cover the end of the draw-in/out guide path 111. Therefore, the cover part 240 is further included to prevent the contaminant of the touch object 10 stained on the touch part 120 from being discharged to the outside through the draw-in/out guide path 111 or prevent the contaminant from coming into contact with a user's body, clothes, and belongings. Then, when the indirect touch apparatus 200 of the present invention is used and then touched by hand or put into a pocket, contamination of the hand or pocket may be more effectively prevented.

For example, the cover part 240 may include a cover member 241 and a slit 242 as illustrated in FIGS. 3 and 4. The cover member 241 may be made of an elastic material and may cover an end of the draw-in/out guide path 111. The slit 242 may be formed in the middle of the cover member 241, and the slit 242 may be opened by pressing the touch part 120 while the touch part 120 is drawn out, and may be contracted and closed by restoring by the self-elasticity of the cover member 241 while the touch part 120 is drawn in.

Therefore, while the touch part 120 is drawn out, the slit 242 of the cover member 241 made of an elastic material is opened so that the touch part 120 may be smoothly drawn out. When the draw-in of the touch part 120 is completed, the slit is closed due to the contraction of the cover member 241 to block the draw-in/out guide path 111, thereby configuring the cover part with a relatively simple configuration.

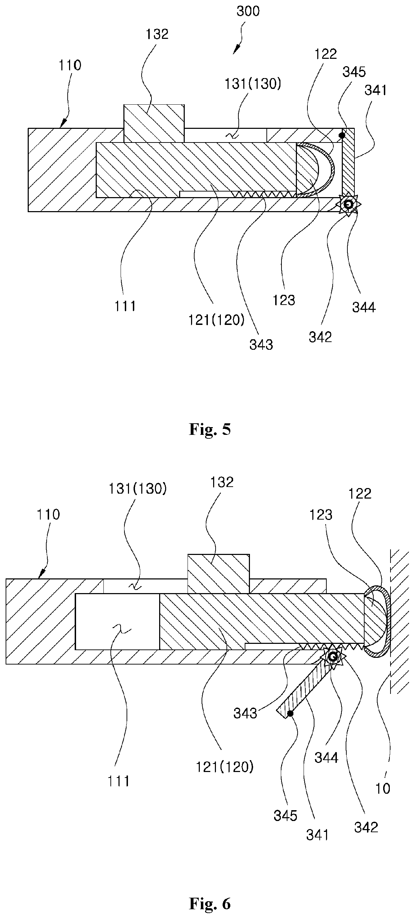

Hereinafter, a direct touch apparatus 300 according to a third embodiment of the present invention will be described with reference to FIGS. 5 and 6.

FIG. 5 is a diagram schematically illustrating an indirect touch apparatus according to a third embodiment of the present invention and FIG. 6 is a diagram schematically illustrating a state in which a touch part of the indirect touch apparatus of FIG. 5 is drawn out.

The indirect touch apparatus according to the third embodiment of the present invention is the same as that of the first embodiment of the present invention described above except that a cover part 340 is further included, as illustrated in FIGS. 5 and 6, and thus, hereinafter, the cover part 340 will be mainly described. For reference, the cover part 340 has a mechanism different from that mentioned in the second embodiment of the present invention described above.

The cover part 340 may include a cover member 341, a pinion 342, and a rack 343, as illustrated in FIGS. 5 and 6. The cover member 341 may be made of a hard material and may be provided rotatably at the end of the draw-in/out guide path 111. The pinion 342 may be provided on a rotation shaft of the cover member 341, and may have, for example, a shape in which a plurality of gear teeth are arranged at intervals along a circumference thereof in concentric circle around the rotation center of the rotation shaft. The rack 343 may be provided on one surface of the touch part 120 so as to be geared to the pinion 342, and for example, may have a shape in which a plurality of gear teeth are arranged at intervals along the length of the touch part 120.

Accordingly, while the touch part 120 is drawn out, the rack 343 provided therein is drawn out together to rotate the pinion 342 in the direction in which the cover member 340 is opened. On the contrary, while the touch part 120 is drawn in, the pinion 342 may be rotated in the direction in which the cover member 341 is closed, so that the cover member 241 may have improved durability as compared with the cover part 240 of the second embodiment of the present invention described above which is opened with respect to the slit 242.

Further, the number of gear teeth of the rack 343 may be determined such that the cover member 341 rotates approximately 90° or more or approximately 180° or more when the cover member 341 is opened while geared to the pinion 342. In particular, when the cover member 341 is opened with a structure that is rotated by 180° or more, the cover member 341 is not elongated in the longitudinal direction of the touch part 120, may be rotated approximately 180° or more at a position vertical to the longitudinal direction of the housing 110, thereby preventing the cover member 341 from interfering with the touch object 10 while the touch part touches the touch object 10.

In addition, the cover part 340 may further include a torsion coil spring 344 as illustrated in FIGS. 5 and 6. The torsion coil spring 344 may be provided on the rotational shaft of the cover member 341, and when the draw-in of the touch part 120 is completed and the rack 343 is disengaged with the pinion 342, the torsion coil spring 344 may apply the rotational force to the cover member 341 in a closing direction. Therefore, even if the gear engagement between the rack 343 and the pinion 342 is momentarily released after the touch part 120 is drawn in and the cover member 341 is closed, the rotational force of the torsion coil spring 344 may be applied in the closing direction to increase the adhesion of the cover member 341 to the end of the housing 110.

In addition, a sealing member 345 such as silicone or rubber for sealing may be provided at a portion of the cover member 341 in contact with the housing 110.

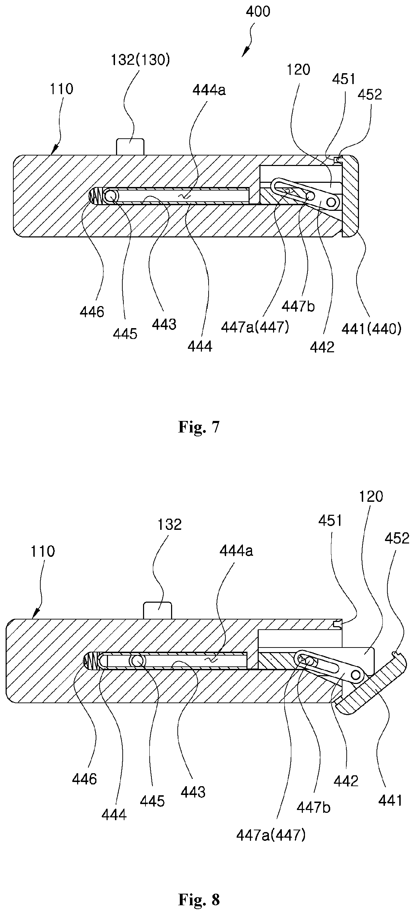

Hereinafter, a direct touch apparatus 400 according to a fourth embodiment of the present invention will be described with reference to FIGS. 7 to 10.

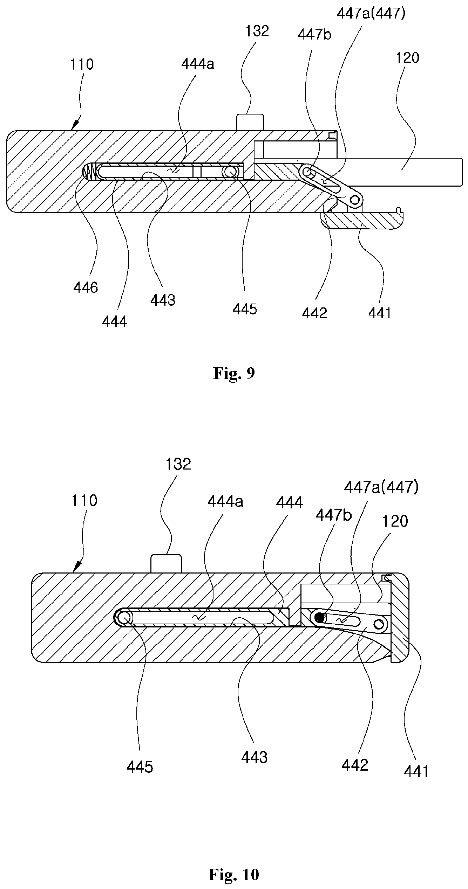

FIG. 7 is a diagram schematically illustrating an indirect touch apparatus according to a fourth embodiment of the present invention, FIG. 8 is a diagram schematically illustrating a state in which a touch part of the indirect touch apparatus of FIG. 7 is drawn out to push a cover member, and FIG. 9 is a diagram schematically illustrating a state in which the touch part of the indirect touch apparatus of FIG. 8 is fully drawn out. FIG. 10 is a diagram schematically illustrating a state in which a front-rear moving rail is pulled by a pulling protrusion while the touch part of the indirect touch apparatus of FIG. 9 is drawn in through the state of FIG. 8.

The indirect touch apparatus 400 according to the fourth embodiment of the present invention is the same as that of the first embodiment of the present invention described above except that a cover part 440 is further included, as illustrated in FIGS. 7 to 10, and thus, hereinafter, the cover part 440 will be mainly described. For reference, the cover part 440 has a mechanism different from that mentioned in the second embodiment of the present invention described above.

The cover part 440, as illustrated in FIGS. 7 to 10, includes a cover member 441, a link member 442, a guide groove 443, a front-rear moving rail 444, and a pull protrusion 445. The cover member 441 may be rotatably provided below the front end of the housing 110, and the link member 442 may have one end rotatably provided on the inner surface of the cover member 441. The guide groove 443 may be elongated on the inner surface of the housing 110 in a longitudinal direction, but may be provided to communicate with the draw-in/out guide path (see 111 in FIG. 2). The front-rear moving rail 444 may be provided to be movable front and rear on the guide groove 443, have the other end of the link member 442 rotatably provided at a front end thereof, and have an front-rear guide hole 444a elongated in a longitudinal direction. The pulling protrusion 445 may be provided to protrude from the side of the touch part 120, may move along the front-rear guide hole 444a while the touch part 120 is drawn in and out by the pressing part 130 as illustrated in FIGS. 8 and 9, and may pull the front-rear moving rail 444 back when the touch part 120 is drawn in by completely pulling the pressing part 130 back as illustrated in FIG. 10.

Further, the cover part 440 may further include an elastic body 446 and a position maintaining part 447 as illustrated in FIGS. 7 to 10. Therefore, the front-rear moving rail 444 retracted back by pulling the pressing part 130 backward (that is, the touch part 120 and the pulling protrusion 445 are retracted) is bounced forward by the restoring force of the elastic body 446 to give a feeling of motion. Even if the front-rear moving rail 444 is moved forward, the position of the link member 442 may be maintained as it is by the position maintaining part 447, that is, the link member 442 is not moved forward, thereby preventing the cover member 441 from opening.

For example, the elastic body 446 may be provided between the rear end of the front-rear moving rail 444 and the rear end of the guide groove 443, and may apply a forward pushing force to the front-rear moving rail 444. As the elastic body 446, a compression coil spring or the like may be used. The position maintaining part 447 may maintain the position of the link member 442 even if the front-rear moving rail 444 is pushed forward by the elastic body 446 due to bouncing or the like.

Further, the position maintaining part 447 may include a push guide hole 447a and a rotation shaft 447b, as illustrated in FIGS. 7 to 10. The pushing guide hole 447a may be elongated in a longitudinal direction thereof in the link member 442. The rotation shaft 447b may be provided at the front end of the front-rear moving rail 444, may penetrate through the push guide hole 447a, and as illustrated in FIGS. 7 and 10, may move along the pushing guide hole 447a when the front-rear moving rail 444 is pushed forward while guiding the rotation of the link member 442.

In addition, as illustrated in FIG. 7, when the cover member 441 is closed, a mounting groove 451 may be formed at the front end of the housing 110 so that the cover member 441 may be maintained in the closed state, and a mounting protrusion 452 may be provided on the inner surface of the cover member 441 so as to be fitted and mounted into the mounting groove 451.

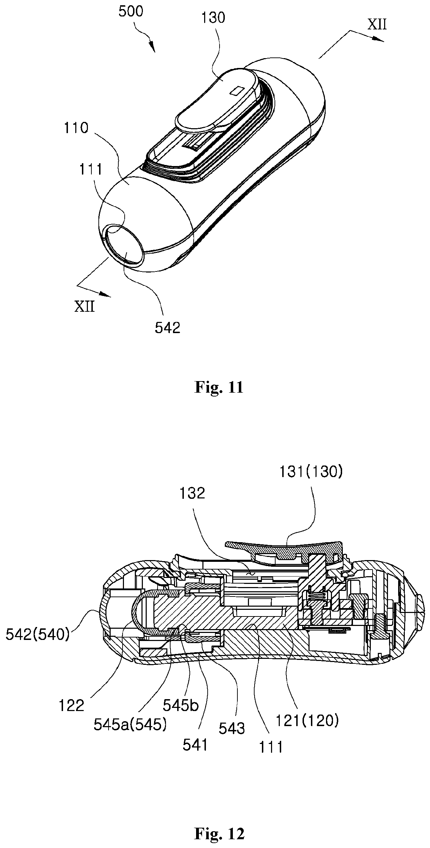

Hereinafter, a direct touch apparatus 500 according to a fourth embodiment of the present invention will be described with reference to FIGS. 11 to 17.

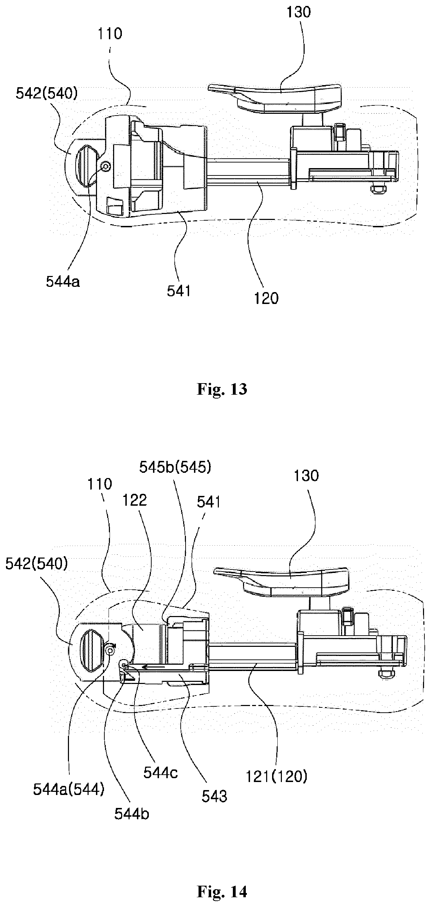

FIG. 11 is a diagram schematically illustrating an indirect touch apparatus according to a fifth embodiment of the present invention. FIG. 12 is a diagram of the indirect touch apparatus of FIG. 11 taken along line XII-XII, FIG. 13 is a side view illustrating the indirect touch apparatus of FIG. 11 from which a housing is removed, and FIG. 14 is a side view illustrating the indirect touch apparatus of FIG. 13 from which a reference member is removed.

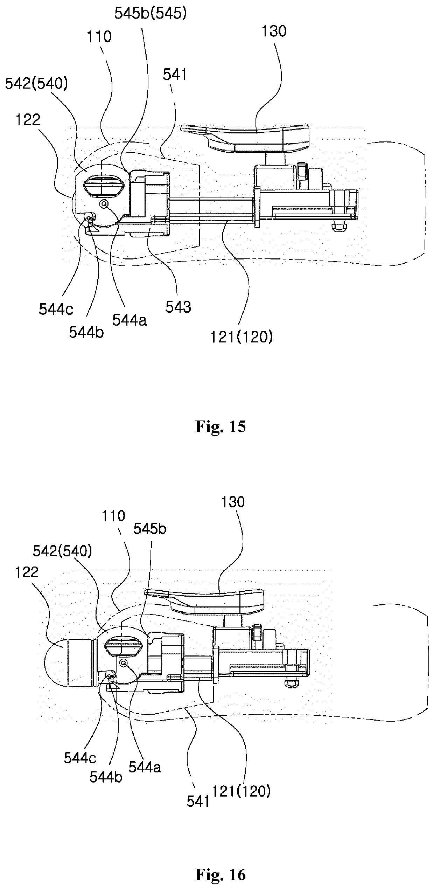

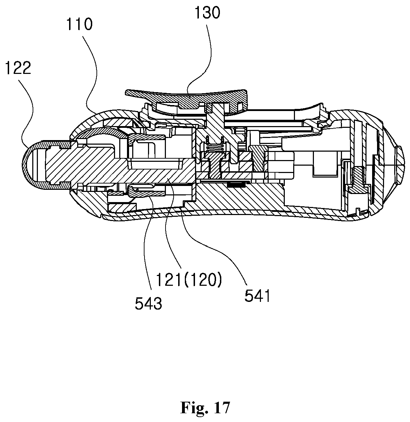

FIG. 15 is a diagram schematically illustrating a state in which a cover part is opened before the touch part comes into contact with the cover part while the touch part is moved in a direction to be drawn out by a pressing part in the indirect touch apparatus of FIG. 14 and FIG. 16 is a diagram schematically illustrating a state in which the touch part is fully drawn out by the pressing part in the indirect touch apparatus of FIG. 15. FIG. 17 is a diagram schematically illustrating a state in which the touch part is fully drawn out in the indirect touch apparatus of FIG. 12.

The indirect touch apparatus 500 according to the fifth embodiment of the present invention is the same as that of the first embodiment of the present invention described above except that a cover part 540 is further included, as illustrated in FIGS. 11 to 17, and thus, hereinafter, the cover part 540 will be mainly described. For reference, the cover part 540 has a mechanism different from that mentioned in the second embodiment of the present invention described above.

While the cover part 540 is provided at the front end of the housing 110, as illustrated in FIGS. 14 and 15, when the touch part 120 is drawn out, the cover part 540 may have a mechanism of opening the end of the draw-in/out guide path 111 in advance with a time lag before coming into contact with the touch part 120 while the touch part 120 is moved in a direction to be drawn out by the pressing part 130. On the contrary, when the touch part 120 is drawn in (see in order to FIG. 14 from FIG 15), the cover part 540 may have a mechanism of closing the end of the draw-in/out guide path with a time lag after the touch part 120 is drawn in.

Therefore, since the end of the touch part 120 does not directly contact the cover part 540 while the cover part 540 is opened or closed, there is no interference between the touch part 120 and the cover part 540 to improve the durability of the touch part 120 and the cover part 540. Since there is no friction with the touch part 120, the cover part 540 may be opened or closed smoothly, and the cover part 540 may always be kept in a clean state even if a contaminant is stained on the end of the touch part 120.

For example, the cover part 540, as illustrated in FIGS. 12 to 17, may include a reference member 541, a cover member 542, a moving member 543, and a motion switching part 544.

The reference member 541 may be fixed to the inner front end of the housing 110 and the cover member 542 may be rotatably provided at the front end of the reference member 541. The moving member 543 may be provided between the touch part 120 and the reference member 541, may be selectively connected to the touch part 120, and may move front and rear together with the touch part 120 when connected to the touch part 120. In addition, the moving distance may be limited by the reference member 541 so as not to be moved any longer when the cover member 542 is opened and closed. The motion switching part 544 may rotate the cover member 542 forward and backward by the front and rear movement of the moving member 543.

Therefore, in a state in which the moving member 543 is connected to the touch part 120 (see FIG. 14), when the touch part 120 moves in the direction to be drawn out by the pressing part 130, the moving member 543 moves together (see arrow in FIG. 14), and the cover member 542 rotatably provided at the front end of the reference member 541 is rotated, so that the end of the draw-in/out guide path 111 may be opened (see FIG. 15). Thereafter, when the touch part 120 is continuously moved in the direction to be drawn out by the pressing part 130, the moving member 543 is placed in a stopped state due to the movement limitation by the reference member 541, and thus, the connection with the touch part 120 may be released and the draw-out of the touch part 120 may be completed (see FIGS. 16 and 17).

Further, the motion switching part 544 may include a cover hinge 544a, a locking groove 544b, and a power transmission protrusion 544c, as illustrated in FIGS. 14 to 16. The cover hinge 544a may rotatably connect the cover member 542 to a front end of the reference member 541. The locking groove 544b may be formed at an edge of the cover member 542 and provided to be eccentric with the cover hinge 544a. The power transmission protrusion 544c may be provided at the front end of the moving member 543 and selectively locked to the locking groove 544b while the moving member 543 moves front and rear to apply a forward and reverse rotational force to the cover member 542.

In addition, as illustrated in FIGS. 14 and 15, the moving member 543 may be selectively connected to the touch part 120 through a connection part 545.

For example, the connection part 545 may include a connection groove 545a and a connection protrusion 545b, as illustrated in FIGS. 12, 14 and 15. The connection groove 545a may be provided on the touch part 120, and the connection protrusion 545b may be provided in the moving member 543 so as to be locked to or removed from the connection groove 545a while the touch part 120 moves front and rear. Therefore, when the moving member 543 is stopped due to the movement limitation by the reference member 541, the connection may be released while the connection protrusion 545b is removed from the connection groove 545a, and when the touch part 120 is moved in the draw-in direction, the connection protrusion 545b of the moving member 543 may be fitted and connected into the connection groove 545a of the touch part. The connection protrusion 545b may obtain a force to be fitted into or removed from the connection groove 545a by self-elasticity.

While this invention has been described in connection with what is presently considered to be practical example embodiments, it is to be understood that the invention is not limited to the disclosed embodiments, but, on the contrary, is intended to cover various modifications and equivalent arrangements included within the spirit and scope of the appended claims.

### Explanation of Reference Numerals and Symbols

| | | | |
|---|---|---|---|
| 10: | Touch object | | |
| 100, 200, 300, 400, 500: | Indirect touch apparatus | | |
| 110: | Housing | 111: | Draw-in/out guide path |
| 120: | Touch part | 121: | Sliding member |
| 122: | Touch member | 123: | Pressure-resistant member |
| 130: | Pressing part | 131: | Knob guide hole |
| 132: | Pressing knob | 240, 340, 440, 540: | Cover part |
| 241, 341, 441: | Cover member | 242: | Slit |
| 342: | Pinion | 343: | Rack |
| 344: | Torsion coil spring | 442: | Link member |
| 443: | Guide groove | 444: | Front-rear moving rail |
| 444a: | Front-rear guide hole | 445: | Pulling protrusion |
| 541: | Reference member | 542: | Cover member |
| 543: | Moving member | 544: | Motion switching part |
| 545: | Connection part. | | |

## Claims

1. An indirect touch apparatus for touching indirectly a touch object, the indirect touch apparatus comprising:
a housing having a draw-in/out guide path concaved in a longitudinal direction;
a touch part slidably provided on the draw-in/out guide path and drawn out to come in contact with the touch object; and
a pressing part drawing in/out the touch part to/from the housing.

2. The indirect touch apparatus of claim 1, further comprising:
a cover part which is provided at a front end of the housing and selectively covers an end of the draw-in/out guide path.

3. The indirect touch apparatus of claim 1, wherein while the cover part is provided at the front end of the housing, the cover part opens the end of the draw-in/out guide path using a draw-out force of the touch part while the touch part is drawn out by the pressing part and closes the end of the draw-in/out guide path while the touch part is drawn in by the pressing part.

4. The indirect touch apparatus of claim 1, wherein while the cover part is provided at the front end of the housing, the cover part opens the end of the draw-in/out guide path in advance with a time lag before coming into contact with the touch part while the touch part is moved in a direction to be drawn out by the pressing part and closes the end of the draw-in/out guide path with a time lag after the touch part is drawn in.

5. The indirect touch apparatus of claim 1, wherein the touch part is made of an antimicrobial material or the antimicrobial material is contained therein or coated on the surface thereof.

6. The indirect touch apparatus of claim 1, wherein the touch part is made of a conductive material or the conductive material is contained therein or coated on the surface thereof.
